# EUROPEAN PATENT APPLICATION

(11) **EP 3 530 985 A1**
(43) Date of publication of application: **28.08.2019**
(21) Application number: 17863438.2
(22) Date of filing: 15.05.2017
(51) Int. Cl.: F16H 25/24, F16H 25/20

(54) **ACTUATOR**

(30) Priority: 24.10.2016 JP 2016207998
(71) Applicant: NSK Ltd., Tokyo 141-8560 (JP)
(72) Inventor: TAGUCHI, Toshifumi, Fujisawa-shi Kanagawa 251-8501 (JP)
(74) Representative: SSM Sandmair
(86) International application number: PCT/JP2017/018136
(87) International publication number: WO 2018/078920

(57) **Abstract**

An actuator includes a frame, a guide rail attached to the frame, a slider guided by the guide rail, a rotational motion-linear motion conversion mechanism that is disposed on a side opposite to the guide rail with the frame interposed therebetween and includes a screw shaft supported by the frame and a nut attached to the screw shaft, and a coupling member that couples the slider and the nut. The coupling member is coupled to the nut via a nut bracket. The coupling member is an annular member along an axial direction of the screw shaft, and includes a slider connection portion that has a plate shape and is fixed to the slider, and a nut bracket connection portion that has a plate shape and is fixed to the nut bracket.

## Description

### Field

The present invention relates to an actuator.

### Background

As devices that discharge liquid in syringes at certain flow rates, actuators have been known that use rotational motion-linear motion conversion mechanisms (ball screws). For example, Patent Literature 1 describes a syringe drive unit that discharges liquid in a syringe at a certain flow rate. The syringe drive unit in Patent Literature 1 includes a guide rail, a screw shaft that is provided in parallel with the guide rail and rotated by a motor, and a slider that moves along the guide rail in accordance with rotation of the screw shaft. In accordance with movement of the slider, liquid in the syringe is discharged at a certain flow rate.

### Citation List

### Patent Literature

Patent Literature 1: Japanese Patent Application Laid-open No. 2010-229926 A

### Summary

### Technical Problem

In order to increase accuracy of flow rate of discharged liquid, it is required that no variation occurs in a movement amount of the slider with respect to a rotation amount of the ball screw. It is thus desirable that the ball screw and the guide rail be perfectly in parallel with each other. The ball screw, however, may not be in parallel with the guide rail in no small degree due to errors in assembling respective members, for example. This may cause variation in the movement amount of the slider with respect to the rotation amount of the ball screw.

In view of the problem described above, the present invention is made and aims to provide an actuator that can prevent variation in a movement amount of a slider with respect to a rotation amount of a rotational motion-linear motion conversion mechanism.

### Solution to Problem

In order to achieve the aim described above, an actuator according to the present invention includes a frame, a guide rail attached to the frame, a slider guided by the guide rail, a rotational motion-linear motion conversion mechanism that is disposed on a side opposite to the guide rail with the frame interposed therebetween and includes a screw shaft supported by the frame and a nut attached to the screw shaft, and a coupling member that couples the slider and the nut. The coupling member is coupled to the nut via a nut bracket. The coupling member is an annular member along an axial direction of the screw shaft and includes a slider connection portion that has a plate shape and is fixed to the slider, and a nut that has a plate shape and is fixed to the nut bracket.

The rotational motion-linear motion conversion mechanism is disposed on the side opposite to the guide rail with the frame interposed therebetween, resulting in a distance from the rotational motion-linear conversion mechanism to the guide rail being larger than that when the rotational motion-linear conversion mechanism and the guide rail are disposed in the same direction with respect to the frame. This larger distance increases the length of the coupling member that couples the slider and the nut, thereby making it easy for the coupling member to be deformed. The coupling member is deformed, thereby making it easy to hold the screw shaft in parallel with the guide rail even if force causing an angle of the screw shaft with respect to the guide rail to be changed acts on the rotational motion-linear motion conversion mechanism. The coupling member functions as a buffering member to prevent a shift in the direction of the screw shaft with respect to the guide rail. The actuator according to the present invention thus can prevent variation in the movement amount of the slider with respect to the rotation amount of the rotational motion-linear motion conversion mechanism.

In addition, in the coupling member, the torsional stiffness around an axis of the screw shaft easily becomes smaller than that around the axis orthogonal to the screw shaft. The coupling member is thus easily deformed in such a direction that a shift of the direction of the screw shaft with respect to the guide rail is prevented, and is not easily tilted (pitching in the coupling member does not easily occur).

As a desirable embodiment of the present invention, it is preferable that the coupling member is disposed with a clearance between the coupling member and the frame.

This makes it hard for the frame to interfere with the coupling member when the coupling member is deformed. The coupling member thus can be easily deformed.

As a desirable embodiment of the present invention, it is preferable that the coupling member has a line symmetrical shape with respect to a straight line serving as a symmetrical axis viewed from the axial direction of the screw shaft, the straight line passing through the guide rail and the screw shaft.

This makes it hard to generate variation in easiness of deformation of the coupling member according to the direction of acted force. As a result, a shift in the direction of the screw shaft with respect to the guide rail is easily prevented.

As a desirable embodiment of the present invention, it is preferable that the nut bracket includes a nut support that has a plate shape and is orthogonal to the screw shaft.

The nut bracket thus receives force in the axial direction of the screw shaft transferred from the nut by the nut support that has a plate shape and is orthogonal to the screw shaft. This makes it hard for the direction of force transferred from the nut bracket to the coupling member to be shifted with respect to the axial direction of the screw shaft.

As a desirable embodiment of the present invention, it is preferable that the nut bracket supports the nut such that the nut is rotatable around an axis orthogonal to the axial direction of the screw shaft.

This prevents stress from occurring in the rotational motion-linear motion conversion mechanism, for example, even when the screw shaft is tilted (pitching of the screw shaft occurs).

As a desirable embodiment of the present invention, it is preferable to further include a plunger drive member that is disposed on a side opposite to the slider with the coupling member interposed therebetween and moves together with the coupling member. It is preferable that a length of the plunger drive member in an X direction is equal to or larger than half of a length of the coupling member in the X direction, the X direction being in parallel with a surface of the frame to which the guide rail is attached and being orthogonal to the axial direction of the screw shaft.

This reinforces the surface to which the plunger drive member is disposed of the coupling member. The torsional stiffness around the axial direction of the screw shaft thus increases in the coupling member. As a result, the tilting (pitching) of the plunger drive member is prevented.

As a desirable embodiment of the present invention, it is preferable that the nut bracket connection portion is in parallel with the slider connection portion.

As a result, in the coupling member, the connection portion connected to the slider and the connection portion connected to the nut bracket are planes in parallel with each other. This makes it easy to perform a process to fix the coupling member and the slider and a process to fix the coupling member and the nut bracket.

As a desirable embodiment of the present invention, it is preferable that the frame includes a base plate in contact with the guide rail and a reinforcing member disposed on a side opposite to the guide rail with the base plate interposed therebetween. This makes it hard for the base plate to be deformed. In addition, a female screw for fixing the guide rail can be provided to the reinforcing member, thereby allowing the guide rail to be more firmly fixed than a case where the reinforcing member is not provided. As a result, the positioning accuracy of the guide rail increases.

### Advantageous Effects of Present Invention

The present invention can provide the actuator that can prevent variation in the movement amount of the slider with respect to the rotation amount of the rotational motion-linear motion conversion mechanism.

### Brief Description of Drawings

FIG. 1 is a perspective view illustrating an actuator according to an embodiment.
FIG. 2 is a front view illustrating the actuator according to the embodiment.
FIG. 3 is a plan view illustrating the actuator according to the embodiment.
FIG. 4 is a left side view illustrating the actuator according to the embodiment.
FIG. 5 is a right side view illustrating the actuator according to the embodiment.
FIG. 6 is a sectional view taken along A-A in FIG. 3.
FIG. 7 is an enlarged view of a periphery of a support unit in FIG. 6.
FIG. 8 is a schematic diagram explaining a state where a coupling member according to the embodiment is deformed.
FIG. 9 is a left side view of an actuator according to a first modification.
FIG. 10 is a B arrow view of FIG. 9.
FIG. 11 is a perspective view illustrating a coupling member according to a second modification.
FIG. 12 is a perspective view illustrating an actuator according to a third modification.
FIG. 13 is a front view illustrating the actuator according to the third modification.
FIG. 14 is a plan view illustrating the actuator according to the third modification.
FIG. 15 is a bottom view illustrating the actuator according to the third modification.
FIG. 16 is a left side view illustrating the actuator according to the third modification.
FIG. 17 is a right side view illustrating the actuator according to the third modification.
FIG. 18 is a sectional view taken along C-C in FIG. 14.

### Description of Embodiment

The following describes an embodiment of the present invention in detail with reference to the accompanying drawings. The content described in the following embodiment does not limit the present invention. The constituent elements described below include elements that the skilled person could easily think of and substantially identical elements. The constituent elements described below can be combined as appropriate.

### Embodiment

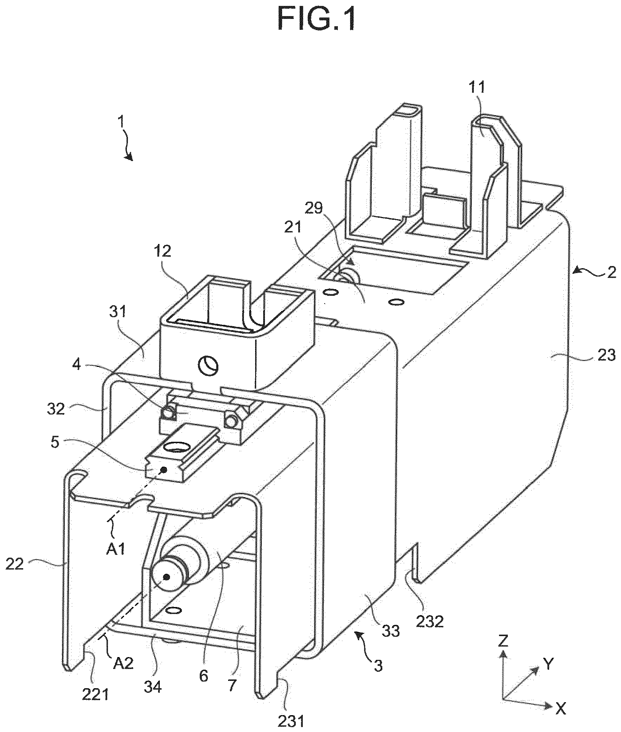

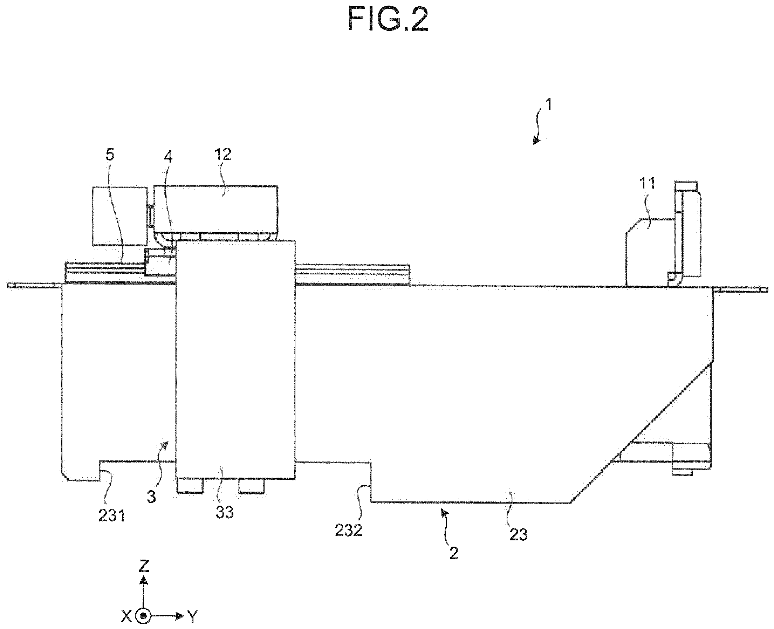

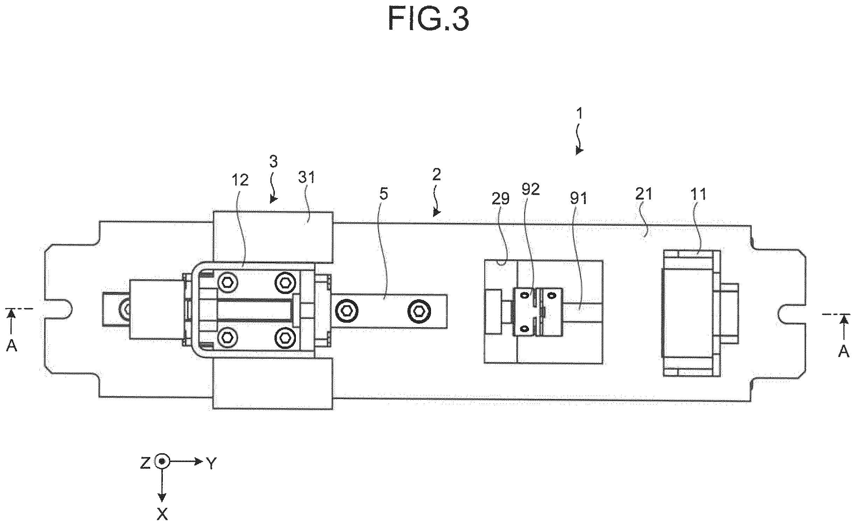

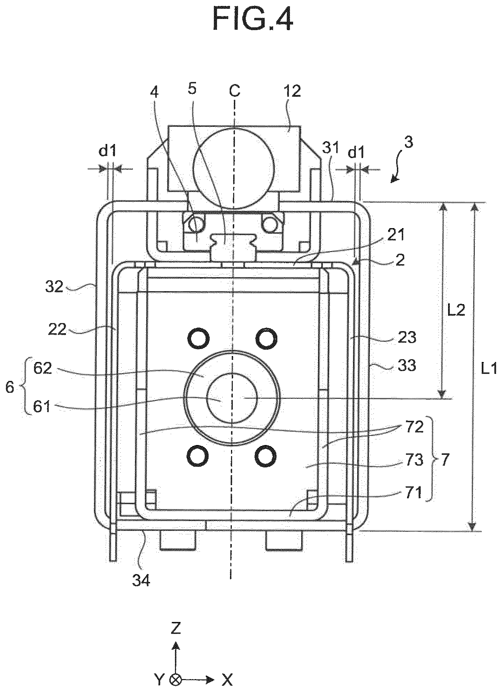

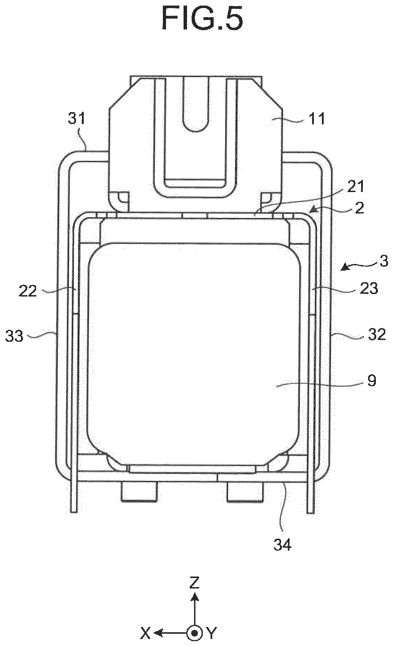

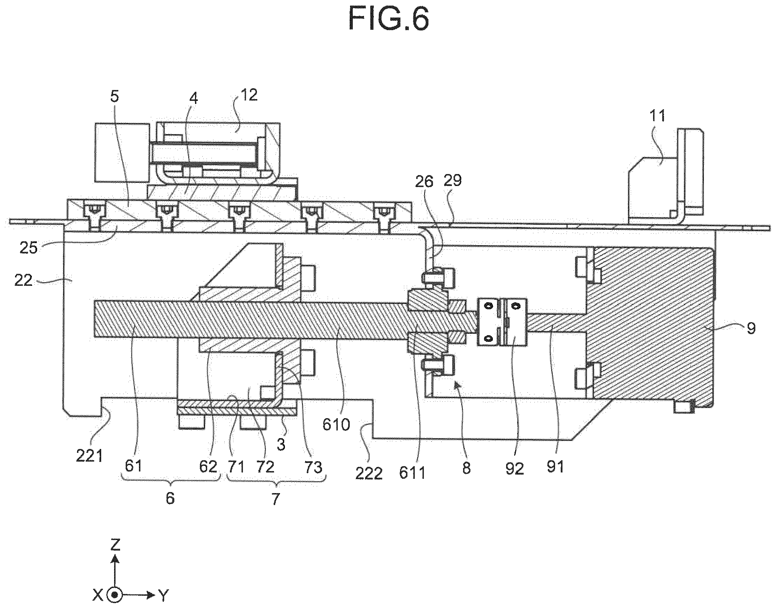

FIG. 1 is a perspective view illustrating an actuator according to an embodiment. FIG. 2 is a front view illustrating the actuator according to the embodiment. FIG. 3 is a plan view illustrating the actuator according to the embodiment. FIG. 4 is a left side view illustrating the actuator according to the embodiment. FIG. 5 is a right side view illustrating the actuator according to the embodiment. FIG. 6 is a sectional view taken along line A-A in FIG. 3. An actuator 1 is a device used for discharging liquid in a syringe at a certain flow rate, for example.

As illustrated in FIGS. 1 to 6, the actuator 1 includes a frame 2, a syringe fixing member 11, a plunger drive member 12, a guide rail 5, a slider 4, a motor 9, a ball screw 6, a nut bracket 7, and a coupling member 3. When liquid in a syringe is discharged using the actuator 1, the syringe is fixed to the syringe fixing member 11 and a plunger is fixed to the plunger drive member 12. The syringe fixing member 11 is fixed to the frame 2. The plunger drive member 12 is fixed to the coupling member 3 that is movable in accordance with rotation of the ball screw 6. The plunger drive member 12 moves in a direction in which the plunger drive member 12 approaches the syringe fixing member 11, resulting in liquid in the syringe being discharged.

The frame 2 is a member that supports the respective members included in the actuator 1. The frame 2 is formed of a metal such as a stainless steel, for example. As illustrated in FIG. 1, the frame 2 includes a base plate 21, and side plates 22 and 23. The base plate 21 and the side plates 22 and 23 are integrally formed by bending a stainless steel plate having a thickness of 1.5 mm, for example. The base plate 21 is a plate-shaped member that is long in a moving direction of the plunger drive member 12, and supports the guide rail 5 and the syringe fixing member 11. The side plates 22 and 23 protrude from both ends in a short side direction of the base plate 21 in a direction orthogonal to the base plate 21. The frame 2 has thus a substantially U-shape when viewed from an axial direction of the ball screw 6. The side plate 22 includes a first facing surface 221 and a second facing surface 222 (refer to FIG. 6). The first facing surface 221 faces one edge of the coupling member 3 in the axial direction of the ball screw 6 while the second facing surface 222 faces the other edge of the coupling member 3 in the axial direction of the ball screw 6. The side plate 23 includes a first facing surface 231 and a second facing surface 232. The first facing surface 231 faces the one edge of the coupling member 3 in the axial direction of the ball screw 6 while the second facing surface 232 faces the other edge of the coupling member 3 in the axial direction of the ball screw 6.

In the following description, an orthogonal coordinate system is used that is composed of an X axis in parallel with the short side direction of the base plate 21, a Y axis in parallel with the axial direction of the ball screw 6, and a Z axis orthogonal to the X axis and the Y axis. In the X direction, the direction on the side plate 22 side of the base plate 21 is defined as the negative X direction while the direction on the side plate 23 side of the base plate 21 is defined as the positive X direction. In the Y direction, the direction on the syringe fixing member 11 side of the plunger drive member 12 is defined as the positive Y direction while the direction opposite to the positive Y direction is defined as the negative Y direction. In the Z direction, the direction in which the side plates 22 and 23 protrude from the base plate 21 is defined as the negative Z direction while the direction opposite to the negative Z direction is defined as the positive Z direction.

The syringe fixing member 11 and the plunger drive member 12 are disposed on the positive Z direction side of the base plate 21. The syringe fixing member 11 is fixed to the base plate 21 by welding, for example. The plunger drive member 12 is fixed to the coupling member 3 by welding, for example.

As illustrated in FIGS. 1 and 3, the base plate 21 is provided with an opening 29. The opening 29 is a rectangular hole, for example. Work for connecting members at the periphery of the motor 9 is done through the opening 29, for example. As illustrated in FIG. 6, to the base plate 21, a back plate 25 and a support unit bracket 26 are attached. The back plate 25 is formed of the same material as the base plate 21, for example. The thickness of the back plate 25 is larger than that of the base plate 21.
The thickness of the back plate 25 is 2 mm, for example. The back plate 25 is fixed to the base plate 21 by welding, for example. The support unit bracket 26 is a plate-shaped member orthogonal to the back plate 25. The support unit bracket 26 is formed integrally with the back plate 25 by bending a stainless steel plate having a thickness of 2 mm. The back plate 25 and the support unit bracket 26 thus form a substantially L shape when viewed from the X direction.

The guide rail 5 is a long member attached to the base plate 21. The guide rail 5 is fixed to a surface of the base plate 21, the surface facing away from the back plate 25. More specifically, the guide rail 5 is fixed to the base plate 21 by fitting fastener members such as bolts passing through the base plate 21 to female screws formed in the back plate 25.

The slider 4 is a member that is guided by the guide rail 5 and movable along the guide rail 5. As illustrated in FIG. 1, the slider 4 holds the guide rail 5 from both sides thereof in the X direction. The slider 4 has therein a plurality of rolling elements in contact with the guide rail 5. The rolling elements are balls, for example, and rotate while in contact with the guide rail 5. The slider 4 thus, can perform straight line motion smoothly along the guide rail 5. The slider 4 and the guide rail 5 form a linear motion guide. More specifically, the multiple rolling elements are circularly disposed and circulate while rotating. The multiple rolling elements form an endless circulation path.

As described above, all of the linear motion guide composed of the slider 4 and the guide rail 5, the syringe fixing member 11, and the plunger drive member 12 are disposed on the positive Z direction side of the base plate 21. The linear motion guide is thus disposed in the vicinity of the syringe, thereby making it possible to prevent tilting (pitching and rolling) of the syringe fixing member 11 due to external force. As a result, accuracy of discharged flow rate of liquid in the syringe increases. The pitching is rotation around the X axis. The rolling is rotation around the Y axis.

The motor 9 is supported by the frame 2 and is provided with a shaft 91. The motor 9 is a stepping motor, for example, and can rotate by a certain angle in accordance with a pulse signal supplied from a control device. The shaft 91 is connected to the ball screw 6 via a coupling 92, for example. The ball screw 6 is a rotational motion-linear motion conversion mechanism that can covert rotational motion into linear motion. The ball screw 6 includes a screw shaft 61 including a main body 610 and a connection portion 611, and a nut 62 attached to the main body 610. Specifically, the shaft 91 is coupled to the connection portion 611 provided at one end of the screw shaft 61 via the coupling 92. The screw shaft 61 has a thread formed on a surface of the main body 610. The connection portion 611 has a smooth surface. A thread formed on an inner wall of the nut 62 is fitted to the thread formed on the main body 610. The outer diameter of the connection portion 611 is smaller than that of the main body 610.

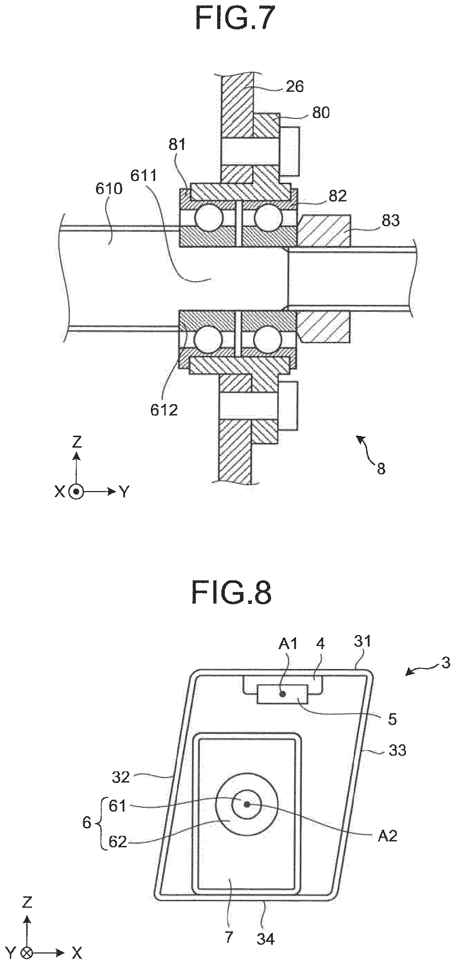

FIG. 7 is an enlarged view of a periphery of a support unit in FIG. 6. As illustrated in FIG. 7, to the support unit bracket 26, a support unit 8 is attached with fastener members such as bolts. The support unit 8 is a member that supports the ball screw 6 in a rotatable manner. The support unit 8 includes a case 80, bearings 81 and 82, and a locking nut 83.

The case 80 is in contact with outer races of the bearings 81 and 82 and supports the bearings 81 and 82.
The bearings 81 and 82 are press-fitted into a hole preliminarily provided to the case 80, for example. As a result, the outer races of the bearings 81 and 82 are fixed to the case 80. The bearings 81 and 82 are juxtaposed along the axial direction of the screw shaft 61. An inner race of the bearing 81 is in contact with a riser 612 between the main body 610 and the connection portion 611 while the inner race of the bearing 82 is in contact with the locking nut 83 attached to the connection portion 611. The bearings 81 and 82 are positioned by the riser 612 and the locking nut 83.

The nut bracket 7 is the member connected to the nut 62 of the ball screw 6. As illustrated in FIG. 6, the nut bracket 7 includes a base 71, a nut support 73, and a reinforcing portion 72. The base 71, the nut support 73, and the reinforcing portion 72 are integrally formed by bending and welding a stainless steel plate, for example.

The base 71 is a plate-shaped member in parallel with the base plate 21, for example. The nut support 73 is a plate-shaped member orthogonal to the Y axis. The nut 62 passes through a hole provided to the nut support 73 and is fixed to the nut support 73 by fastener members such as bolts. The nut bracket 7 thus moves integrally with the nut 62 as the nut 62 moves. The reinforcing portion 72 is a plate-shaped member orthogonal to both of the base 71 and the nut support 73, and is connected to both of the base 71 and the nut support 73. The reinforcing portion 72 prevents the nut support 73 from being tilted in such a manner that the nut support 73 changes an angle with respect to the base 71. This structure makes it hard for the nut support 73 to be deformed even when external force is applied to the nut support 73, thereby allowing a posture of the nut 62 to be easily maintained.

The coupling member 3 couples the slider 4 and the nut 62. Specifically, the coupling member 3 couples the slider 4 to the nut 62 via the nut bracket 7. The coupling member 3 is an annular member along the Y direction, for example, as illustrated in FIG. 1. The coupling member 3 includes a slider connection portion 31, a first circumventing portion 32, a second circumventing portion 33, and a nut bracket connection portion 34. The annular member along the Y direction means that the axial direction of the annular member is along the Y direction. The term is used as the same meaning as that described above in the following description. The slider connection portion 31, the first circumventing portion 32, the second circumventing portion 33, and the nut bracket connection portion 34 are integrally formed by bending and welding a stainless steel plate, for example.

The slider connection portion 31 is a plate-shaped member in parallel with the base plate 21. In the X direction, the slider connection portion 31 is longer than the base plate 21. In other words, when viewed from the Z direction, each of both ends of the slider connection portion 31 in the X direction is located outside the corresponding one of both ends of the base plate 21 in the X direction. The slider connection portion 31 is fixed to the slider 4 by fastener members such as bolts. The coupling member 3 is thus guided by the guide rail 5 together with the slider 4. Specifically, the slider 4 is fixed on the negative Z direction side of the slider connection portion 31. On the positive Z direction side of the slider connection portion 31, the plunger drive member 12 is fixed.

Each of the first circumventing portion 32 and the second circumventing portion 33 is a plate-shaped member orthogonal to the slider connection portion 31. The first circumventing portion 32 and the second circumventing portion 33 protrude from both ends of the slider connection portion 31 in the negative Z direction. The first circumventing portion 32 is located on the negative X direction side of the side plate 22 of the frame 2. The first circumventing portion 32 faces the side plate 22 with a clearance therebetween that has a width d1. The width d1 is about 1 mm to 2 mm, for example. The second circumventing portion 33 is located on the positive X direction side of the side plate 23 of the frame 2. The second circumventing portion 33 faces the side plate 23 with a clearance therebetween that has the width d1. As illustrated in FIG. 4, a length L1 of each of the first circumventing portion 32 and the second circumventing portion 33 in the Z direction is longer than a length L2 between the slider connection portion 31 to the ball screw 6. The ends opposite to those on the slider connection portion 31 side of the first circumventing portion 32 and the second circumventing portion 33 are located on the negative Z direction side of the ball screw 6.

If the first circumventing portion 32 and the second circumventing portion 33 do not extend outside but extend inside the frame 2, i.e., the first circumventing portion 32 and the second circumventing portion 33 pass through a slit provided to the base plate 21, for example, torsional stiffness of the base plate 21 is reduced. Partial loss of sectional area occurs corresponding to the slit through which the first circumventing portion 32 and the second circumventing portion 33 pass in the base plate 21, thereby reducing the torsional stiffness. As a result, the base plate 21 is easily deformed by external force. This arises a problem in that positioning accuracy of the guide rail 5 is reduced or a life-span is reduced due to stresses occurring in the guide rail 5 and the ball screw 6, for example. In contrast, in the embodiment, the first circumventing portion 32 and the second circumventing portion 33 circumvent the frame 2, thereby preventing the base plate 21 from being deformed. In addition, processing steps for making the base plate 21 are reduced, thereby making it easy to manufacture the frame 2.

The nut bracket connection portion 34 is a plate-shaped member in parallel with the slider connection portion 31, and connects the first circumventing portion 32 and the second circumventing portion 33. The coupling member 3 thus has a substantially oblong shape when viewed from the Y direction. More specifically, when viewed from the Y direction, the coupling member 3 has a substantially oblong shape having short sides along the X direction and long sides along the Z direction. In other words, when viewed from the Y direction, the coupling member 3 has a line symmetrical shape with respect to a straight line C (refer to FIG. 4) serving as the symmetrical axis. The straight line C passes through the slider 4 and the screw shaft 61. The nut bracket connection portion 34 is fixed to the base 71 of the nut bracket 7 by fastener members such as bolts. The coupling member 3 thus moves integrally with the nut bracket 7 as the nut 62 moves.

As illustrated in FIGS. 1 and 6, one edge of the nut bracket connection portion 34 faces the first facing surfaces 221 and 231 while the other edge of the nut bracket connection portion 34 faces the second facing surfaces 222 and 232. With the movement of the nut 62, the nut bracket connection portion 34 moves in the negative Y direction by a certain amount, resulting in the nut bracket connection portion 34 being in contact with the first facing surfaces 221 and 231. In this way, a limit position to which the coupling member 3 can move in the negative Y direction is defined. With the movement of the nut 62, the nut bracket connection portion 34 moves in the positive Y direction by a certain amount, resulting in the nut bracket connection portion 34 being in contact with the second facing surfaces 222 and 232. In this way, a limit position to which the coupling member 3 can move in the positive Y direction is defined. The limit positions prevent the nut 62 from dropping off from the screw shaft 61.

When the motor 9 operates, the shaft 91 rotates, thereby rotating the screw shaft 61 of the ball screw 6. With the rotation of the screw shaft 61, the nut 62 fitting the thread of the screw shaft 61 moves in the Y direction. The nut bracket 7, the coupling member 3, and the slider 4 thus move in the Y direction together with the nut 62. A movement amount of the plunger drive member 12 in the Y direction is equal to that of the slider 4 in the Y direction. In order to increase accuracy of flow rate of liquid discharged from the syringe provided to the actuator 1, the movement amount of the slider 4 needs to be highly accurately controlled. For highly accurate control, it is desirable that an axis A1 of the guide rail 5 and an axis A2 of the screw shaft 61 be in parallel with each other. The axes A1 and A2 are illustrated in FIG. 1. It is, however, not easy to constantly hold the axes A1 and A2 in parallel with each other. For example, the axis A1 is not in parallel with the axis A2 in no small degree due to errors in assembling respective members to the frame 2. With the movement of the nut 62, the screw shaft 61 is oscillated in no small degree to the support unit 8 serving as a fulcrum. As a result, the axis A1 may not be in parallel with the axis A2. An angle of the axis A2 with respect to the axis A1 may change with time. This may cause variation in the movement amount of the slider 4 with respect to the rotation amount of the ball screw 6.

If the guide rail 5, the slider 4, and the ball screw 6 are disposed close to one another and the ball screw 6 is supported such that the screw shaft 61 does not oscillate, the angle of the axis A1 with respect to the axis A2 is prevented from being changed with time, but stresses may continue to occur in the guide rail 5, the slider 4, and the ball screw 6. These stresses hinder sooth movement of the slider 4. As a result, variation may occur in the movement amount of the slider 4 with respect to the rotation amount of the ball screw 6.

In contrast, in the actuator 1 according to the embodiment, the coupling member 3 is deformed, thereby preventing variation in the movement amount of the slider 4 with respect to the rotation amount of the ball screw 6. FIG. 8 is a schematic diagram explaining a state where the coupling member according to the embodiment is deformed. FIG. 8 is a diagram when the actuator 1 is viewed from direction toward the positive Y direction. FIG. 8 exaggerates the deformation of the coupling member 3 for explanatory purpose. The deformation of the coupling member 3 illustrated in FIG. 8 differs from the practical deformation thereof in some cases.

Even if force causing an angle of the axis A1 with respect to the axis A2 to be changed acts on the ball screw 6, the coupling member 3 is deformed and makes it easy to hold the axes A1 and A2 in parallel with each other. Specifically, as illustrated in FIG. 8, the coupling member 3 is deformed in such a manner that the ends of the first circumventing portion 32 and the second circumventing portion 33 in the Z direction shift in the X direction. As described above, clearances are each provided between the first circumventing portion 32 and the side plate 22 and between the second circumventing portion 33 and the side plate 23, thereby allowing the coupling member 3 to be easily deformed. The coupling member 3 is deformed and makes it hard to cause stress in the guide rail 5, the slider 4, and the ball screw 6. The life-spans of the guide rail 5, the slider 4, and the ball screw 6 are thus elongated.

In order to cause the coupling member 3 to be easily deformed as illustrated in FIG. 8, the torsional stiffness around the Y axis is preferably smaller than that around the X axis in the coupling member 3, for example. In the embodiment, the torsional stiffness around the Y axis is smaller than that around the axis orthogonal to the Y axis in the coupling member 3 because the coupling member 3 has an annular shape along the Y direction. The torsional stiffness of a certain member around a certain axis is expressed as G × J/L where G is the modulus of transverse elasticity of the member, J is the torsion constant of the member, and L is the length in the axial direction of the member.

The coupling member 3 is not always required to have the shape described above. The coupling member 3 may not be an annular member. For example, a slit from one end to the other end in the Y direction may be provided to the slider connection portion 31 or the nut bracket connection portion 34. A slit from one end to the other end in the Y direction may be provided to both of the slider connection portion 31 and the nut bracket connection portion 34. The coupling member 3 may not have a line symmetrical shape with respect to a straight line, which serves as the symmetrical axis, in parallel with the Z axis. For example, either the first circumventing portion 32 or the second circumventing portion 33 may not be included in the coupling member 3. As a result, the coupling member 3 may have a substantially U shape when viewed from the Y direction. The length L1 illustrated in FIG. 4 may not be always required to be longer than the distance L2. The length L1 is preferably larger than the distance L2 from point of view that the coupling member 3 is easily deformed by force in the X direction.

The frame 2 may not be always required to include the side plates 22 and 23. The frame 2 may include at least the base plate 21. If the frame 2 does not include the side plates 22 and 23, the width d1 illustrated in FIG. 4 means the width of the clearance between the first circumventing portion 32 and the base plate 21 or the width of the clearance between the second circumventing portion 33 and the base plate 21.

As described above, the actuator 1 according to the embodiment includes the frame 2, the guide rail 5 attached to the frame 2, the slider 4 guided by the guide rail 5, the rotational motion-linear motion conversion mechanism (ball screw 6) that is disposed on the side opposite to the guide rail 5 with the frame 2 interposed therebetween and includes the screw shaft 61 supported by the frame 2 and the nut 62 attached to the screw shaft 61, and the coupling member 3 that couples the slider 4 and the nut 62.

The ball screw 6 is disposed on the side opposite to the guide rail 5 with the frame 2 interposed therebetween, resulting in the distance from the ball screw 6 to the guide rail 5 being larger than that when the ball screw 6 and the guide rail 5 are disposed in the same direction with respect to the frame 2. This increases the length of the coupling member 3 that couples the slider 4 and the nut 62, thereby making it easy for the coupling member 3 to be deformed. The coupling member 3 is deformed, thereby making it easy to hold the screw shaft 61 in parallel with the guide rail 5 even if force causing an angle of the screw shaft 61 with respect to the guide rail 5 to be changed acts on the ball screw 6. The coupling member 3 functions as a buffering member to prevent a shift in the direction of the screw shaft 61 with respect to the guide rail 5. The actuator 1 according to the embodiment thus can prevent variation in the movement amount of the slider 4 with respect to the rotation amount of the rotational motion-linear motion conversion mechanism (ball screw 6).

In the actuator 1 according to the embodiment, the coupling member 3 is disposed with a clearance with respect to the frame 2. This structure makes it hard for the frame 2 to interfere with the coupling member 3 when the coupling member 3 is deformed. The coupling member 3 thus can be easily deformed.

In the actuator 1 according to the embodiment, the coupling member 3 has a line symmetrical shape with respect to the straight line C serving as the symmetrical axis when viewed from the axial direction (Y direction) of the screw shaft 61, the straight line C passing through the guide rail 5 and the screw shaft 61. This structure makes it hard to generate variation in easiness of deformation of the coupling member 3 according to the direction of acted force. As a result, a shift in the direction of the screw shaft 61 with respect to the guide rail 5 is more easily prevented.

In the actuator 1 according to the embodiment, the coupling member 3 is an annular member along the axial direction (Y direction) of the screw shaft 61. In the coupling member 3, this structure makes it easy for the torsional stiffness around the axis (Y axis) of the screw shaft 61 to be smaller than that around the axis orthogonal to the Y axis. The coupling member 3 is thus easily deformed in such a direction that a shift of the direction of the screw shaft 61 with respect to the guide rail 5 is prevented, and is not easily tilted (pitching of the coupling member 3 does not easily occur).

In the actuator 1 according to the embodiment, the coupling member 3 is coupled to the nut 62 via the nut bracket 7. The nut bracket 7 includes the nut support 73 that has a plate shape and is orthogonal to the screw shaft 61. The nut bracket 7 thus receives force in the axial direction (Y direction) of the screw shaft 61 transferred from the nut 62 by the nut support 73 that has a plate shape and is orthogonal to the screw shaft 61. This structure makes it hard for the direction of force transferred from the nut bracket 7 to the coupling member 3 to be shifted with respect to the axial direction (Y direction) of the screw shaft 61.

In the actuator 1 according to the embodiment, the coupling member 3 is an annular member along the axial direction (Y direction) of the screw shaft 61, and includes the slider connection portion 31 that has a plate shape and is fixed to the slider 4, and the nut bracket connection portion 34 that is fixed to the nut bracket 7 and has a plate shape in parallel with the slider connection portion 31. In the coupling member 3, the connection portion connected to the slider 4 and the connection portion connected to the nut bracket 7 are planes in parallel with each other. This structure makes it easy to perform a process to fix the coupling member 3 and the slider 4 and a process to fix the coupling member 3 and the nut bracket 7.

In the actuator 1 according to the embodiment, the frame 2 includes the base plate 21 in contact with the guide rail 5 and the reinforcing member (back plate 25) disposed on the side opposite to the guide rail 5 with the base plate 21 interposed therebetween. This structure makes it hard for the base plate 21 to be deformed. In addition, female screws for fixing the guide rail 5 can be provided to the reinforcing member, thereby allowing the guide rail 5 to be more firmly fixed than a case where the reinforcing member is not provided. As a result, the positioning accuracy of the guide rail 5 increases.

### First modification

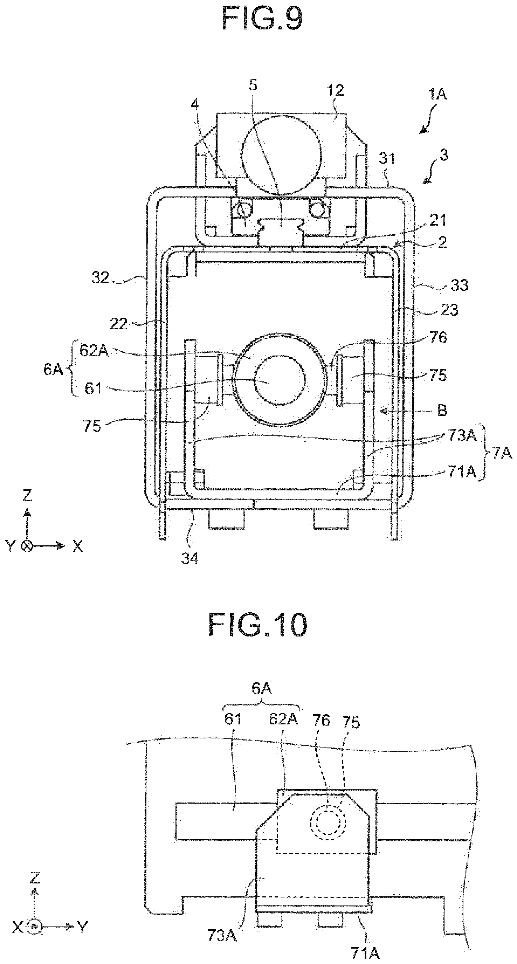

FIG. 9 is a left side view of an actuator according to a first modification. FIG. 10 is a B arrow view of FIG. 9. As illustrated in FIGS. 9 and 10, in the first modification, a ball screw 6A different from the ball screw 6 and a nut bracket 7A different from the nut bracket 7 are provided. The same constituent elements as described in the embodiment are labeled with the same numerals and duplicated descriptions thereof are omitted.

As illustrated in FIGS. 9 and 10, the ball screw 6A includes the screw shaft 61 and a nut 62A. The nut 62A is a cylindrical member, for example. The thread formed on the inner wall of the nut 62A fits the thread formed on the main body 610.

The nut bracket 7A is the member that supports the nut 62A such that the nut 62A can rotate around the X axis. As illustrated in FIGS. 9 and 10, the nut bracket 7A includes a base 71A and two nut supports 73A. The base 71A and the nut supports 73A are integrally formed by bending a stainless steel plate, for example.

The base 71A is a plate-shaped member in parallel with the base plate 21, and is connected to the nut bracket connection portion 34. The nut support 73A is a plate-shaped member orthogonal to the X axis. The nut support 73A includes a bearing 75 and a trunnion 76. The bearing 75 is fixed to the nut support 73A. One end of the trunnion 76 is press-fitted into the inner race of the bearing 75, for example. The other end of the trunnion 76 is fixed to the nut 62A. The nut bracket 7A thus can support the nut 62A such that the nut 62A can rotate around the X axis.

As described above, in an actuator 1A according to the first modification, the coupling member 3 is coupled to the nut 62A via the nut bracket 7A. The nut bracket 7A supports the nut 62A such that the nut 62A can rotate around the X axis. This structure prevents stress from occurring in the ball screw 6A, for example, even when the screw shaft 61 is tilted (pitching of the screw shaft 61 occurs).

### Second modification

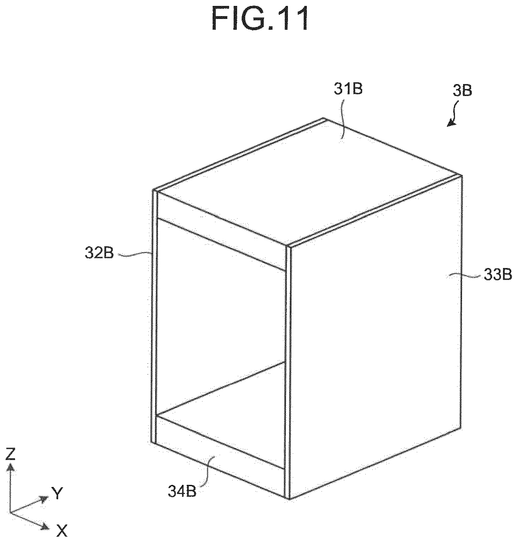

FIG. 11 is a perspective view illustrating a coupling member according to a second modification. In the second modification, a coupling member 3B different from the coupling member 3 is provided. The same constituent elements as described in the embodiment are labeled with the same numerals and duplicated descriptions thereof are omitted.

The coupling member 3B couples the slider 4 and the nut 62. Specifically, the coupling member 3B couples the slider 4 to the nut 62 via the nut bracket 7. The coupling member 3B is an annular member along the Y direction, for example, as illustrated in FIG. 11. The coupling member 3B includes a slider connection portion 31B, a first circumventing portion 32B, a second circumventing portion 33B, and a nut bracket connection portion 34B. The slider connection portion 31B, the first circumventing portion 32B, the second circumventing portion 33B, and the nut bracket connection portion 34B are connected by welding, for example.

The slider connection portion 31B is a plate-shaped member in parallel with the base plate 21. The slider connection portion 31B is a metallic plate such as a stainless steel plate. In the X direction, the slider connection portion 31B is longer than the base plate 21. When viewed from the Z direction, each of both ends of the slider connection portion 31B in the X direction is located outside the corresponding one of both ends of the base plate 21 in the X direction. The slider connection portion 31B is fixed to the slider 4 by fastener members such as bolts. The coupling member 3B is thus guided by the guide rail 5 together with the slider 4. Specifically, the slider 4 is fixed to the slider connection portion 31B on the negative Z direction side of the slider connection portion 31B. On the positive Z direction side of the slider connection portion 31B, the plunger drive member 12 is fixed.

Each of the first circumventing portion 32B and the second circumventing portion 33B is a plate-shaped member orthogonal to the slider connection portion 31B. The first circumventing portion 32B and the second circumventing portion 33B protrude from both ends of the slider connection portion 31B in the negative Z direction. Each of the first circumventing portion 32B and the second circumventing portion 33B is a metallic plate such as a stainless steel plate having a thickness smaller than that of the slider connection portion 31B, for example. The first circumventing portion 32B is located on the negative X direction side of the side plate 22. The first circumventing portion 32B faces the side plate 22 with a clearance therebetween that has the width d1 (refer to FIG. 4). The second circumventing portion 33B is located on the positive X direction side of the side plate 23. The second circumventing portion 33 faces the side plate 23 with a clearance therebetween that has the width d1. In the same manner as the first circumventing portion 32 and the second circumventing portion 33 illustrated in FIG. 4, the length of each of the first circumventing portion 32B and the second circumventing portion 33B in the Z direction is longer than the distance from the slider connection portion 31B to the ball screw 6. The ends opposite to those on the slider connection portion 31B side of the first circumventing portion 32B and the second circumventing portion 33B are located on the negative Z direction side of the ball screw 6.

The nut bracket connection portion 34B is a plate-shaped member in parallel with the slider connection portion 31B, and connects the first circumventing portion 32 and the second circumventing portion 33. The coupling member 3B thus has a substantially oblong shape when viewed from the Y direction. More specifically, when viewed from the Y direction, the coupling member 3B has a substantially oblong shape having short sides along the X direction and long sides along the Z direction. The nut bracket connection portion 34B is a metallic plate such as a stainless steel plate having the same thickness as the slider connection portion 31B, for example. In other words, when viewed from the Y direction, the coupling member 3B has a line symmetrical shape with respect to a straight line serving as the symmetrical axis, the straight line passing through the slider 4 and the screw shaft 61. The nut bracket connection portion 34B is fixed to the base 71 of the nut bracket 7 by fastener members such as bolts.
The coupling member 3B thus moves integrally with the nut bracket 7 as the nut 62 moves.

As described above, in the second modification, the thickness of each of the first circumventing portion 32B and the second circumventing portion 33B is smaller than that of each of the slider connection portion 31B and the nut bracket connection portion 34B. As a result, the first circumventing portion 32B and the second circumventing portion 33B having a thinner thickness reduce the torsional stiffness around the Y axis in the whole of the coupling member 3B while the torsional stiffness of the slider connection portion 31B and the nut bracket connection portion 34B is maintained. The titling (pitching) of the slider 4 and nut bracket 7 is thus prevented.

The slider connection portion 31B, the first circumventing portion 32B, the second circumventing portion 33B, and the nut bracket connection portion 34B may not be always required to be connected by welding. For example, pins provided to the slider connection portion 31B and the nut bracket connection portion 34B fit depressed portions provided to the first circumventing portion 32B and the second circumventing portion 33B, thereby achieving positioning. Thereafter, the slider connection portion 31B, the first circumventing portion 32B, the second circumventing portion 33B, and the nut bracket connection portion 34B are fastened by bolts, for example.

### Third modification

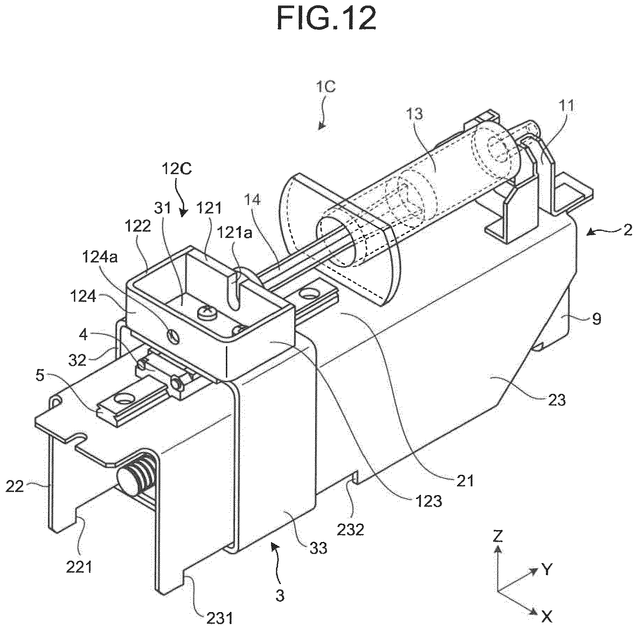

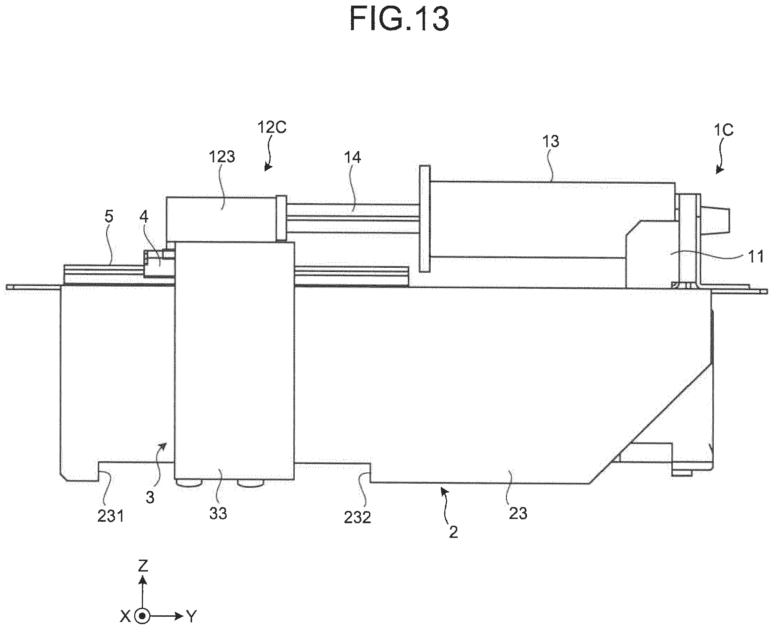

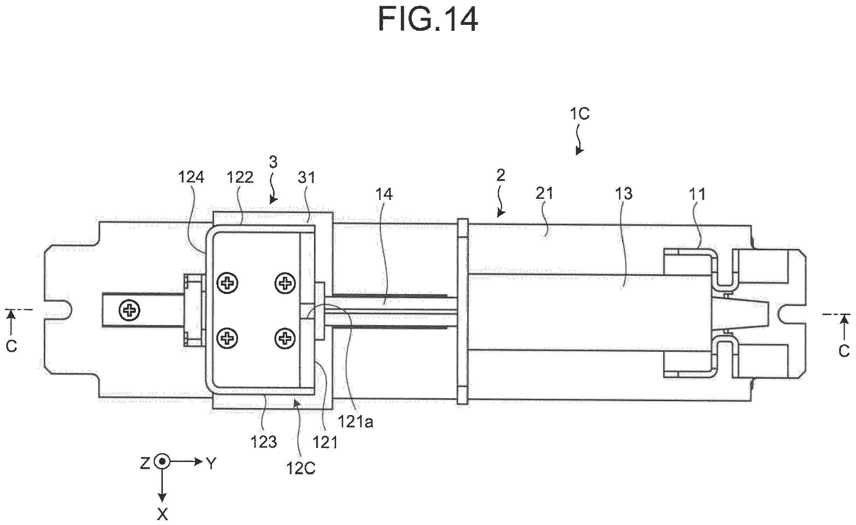

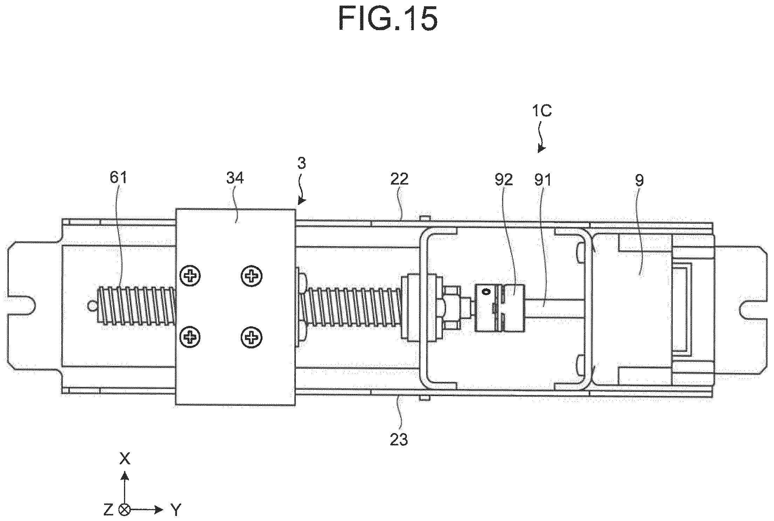

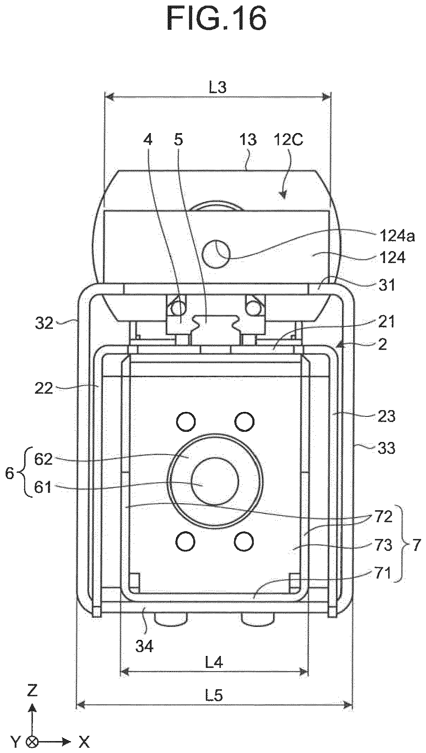

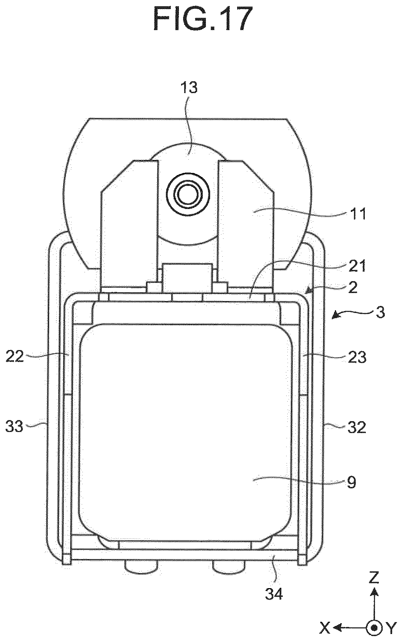

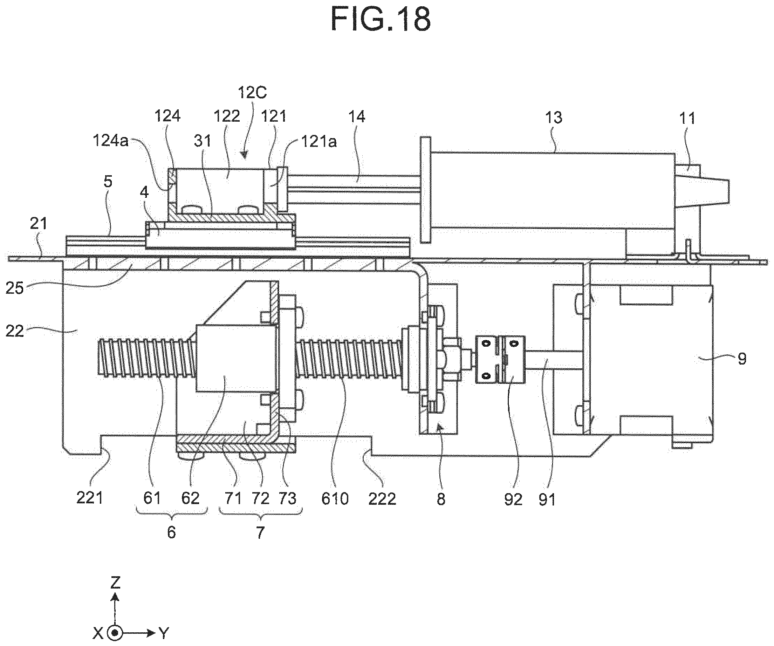

FIG. 12 is a perspective view illustrating an actuator according to a third modification. FIG. 13 is a front view illustrating the actuator according to the third modification. FIG. 14 is a plan view illustrating the actuator according to the third modification. FIG. 15 is a bottom view illustrating the actuator according to the third modification. FIG. 16 is a left side view illustrating the actuator according to the third modification. FIG. 17 is a right side view illustrating the actuator according to the third modification. FIG. 18 is a sectional view taken along C-C in FIG. 14. In FIG. 18, a part of the structure is illustrated as a front view. An actuator 1C according to the third modification includes a plunger drive member 12C different from the plunger drive member 12. The same constituent elements as described in the embodiment are labeled with the same numerals and duplicated descriptions thereof are omitted.

As illustrated in FIG. 12, when liquid in a syringe 13 is discharged using the actuator 1C, the syringe 13 is fixed to the syringe fixing member 11 and a plunger 14 is fixed to the plunger drive member 12C. The plunger drive member 12C is fixed to the coupling member 3 that is movable in accordance with rotation of the ball screw 6. The plunger drive member 12C moves in a direction in which the plunger drive member 12C approaches the syringe fixing member 11, resulting in liquid in the syringe 13 being discharged.

The plunger drive member 12C is disposed on the positive Z direction side of the slider connection portion 31. The plunger drive member 12C is an annular member an axial direction of which is along the Z direction. When viewed from the Z direction, the outer periphery of the plunger drive member 12C has a substantially oblong shape. As illustrated in FIG. 12, the plunger drive member 12C includes a plunger contact portion 121, a facing portion 124, a first side surface portion 122, and a second side surface portion 123.

The plunger contact portion 121 is a plate-shaped member orthogonal to the Y direction, and is in contact with the plunger 14 when the syringe 13 and the plunger 14 are attached to the actuator 1C. The plunger contact portion 121 is fixed to the slider connection portion 31 by welding, for example. The plunger contact portion 121 is provided with a slit 121a extending in the Z direction at the center in the X direction.

The facing portion 124 is a plate-shaped member in parallel with the plunger contact portion 121. The length of the facing portion 124 in the X direction is substantially equal to that of the plunger contact portion 121 in the X direction. The facing portion 124 has a hole 124a at the center in the X direction. The hole 124a overlaps with the slit 121a when viewed from the Y direction.

The first side surface portion 122 and the second side surface portion 123 are plate-shaped members orthogonal to the X direction. The first side surface portion 122 and the second side surface portion 123 are integrated with the facing portion 124, for example. The facing portion 124, the first side surface portion 122, and the second side surface portion 123 are formed by bending a single plate. The length of the second side surface portion 123 in the Y direction is substantially equal to that of the first side surface portion 122 in the Y direction. The facing portion 124, the first side surface portion 122, and the second side surface portion 123 are fixed to the slider connection portion 31 by welding, for example. The ends of the first side surface portion 122 and the second side surface portion 123 in the positive Y direction are fixed to the plunger contact portion 121.
The lengths in the Z direction of the plunger contact portion 121, the facing portion 124, the first side surface portion 122, and the second side surface portion 123 are substantially equal.

In the plunger drive member 12C according to the third modification, the length thereof in the X direction differs from that of the plunger drive member 12 according to the embodiment. As illustrated in FIG. 16, a length L3 of the plunger drive member 12C in the X direction is equal to or larger than half of a length L5 of the coupling member 3 in the X direction and equal to or smaller than the length L5, for example. The length L3 is equal to or larger than a length L4 of the nut bracket 7 in the X direction, for example. In other words, the length L3 is the length of each of the plunger contact portion 121 and the facing portion 124 in the X direction.

As described above, the actuator 1C according to the third modification includes the plunger drive member 12C. The plunger drive member 12C is disposed on the side opposite to the slider 4 with the coupling member 3 interposed therebetween and moves together with the coupling member 3. The length L3 of the plunger drive member 12C in the X direction is equal to or larger than half of the length L5 of the coupling member 3 in the X direction where the X direction is the direction that is in parallel with the surface (base plate 21) of the frame 2 to which the guide rail 5 is attached and is orthogonal to the axial direction of the screw shaft 61. This structure reinforces the surface (slider connection portion 31) to which the plunger drive member 12C is disposed of the coupling member 3. The torsional stiffness around the axial direction (Y axis) of the screw shaft 61 thus increases in the coupling member 3. As a result, the tilting (pitching) of the plunger drive member 12C is prevented.

### Reference Signs List

1, 1A, 1C actuator
11 syringe fixing member
12, 12C plunger drive member
121 plunger contact portion
121a slit
122 first side surface portion
123 second side surface portion
124 facing portion
124a hole
13 syringe
2 frame
21 base plate
22, 23 side plate
221, 231 first facing surface
222, 232 second facing surface
25 back plate
26 support unit bracket
29 opening
3, 3B coupling member
31, 31B slider connection portion
32, 32B first circumventing portion
33, 33B second circumventing portion
34, 34B nut bracket connection portion
4 slider
5 guide rail
6, 6A ball screw (rotational motion-linear motion conversion mechanism)
61 screw shaft
610 main body
611 connection portion
612 riser
62, 62A nut
7, 7A nut bracket
71, 71A base
72 reinforcing portion
73, 73A nut support
75 bearing
76 trunnion
8 support unit
80 case
81, 82 bearing
83 locking nut
9 motor
91 shaft
92 coupling
A1, A2 axis
C straight line

## Claims

1. An actuator, comprising:
a frame;
a guide rail that is attached to the frame;
a slider that is guided by the guide rail;
a rotational motion-linear motion conversion mechanism that is disposed on a side opposite to the guide rail with the frame interposed therebetween and includes a screw shaft supported by the frame and a nut attached to the screw shaft; and
a coupling member that couples the slider and the nut,
wherein
the coupling member is coupled to the nut via a nut bracket, and
the coupling member is an annular member along an axial direction of the screw shaft, and includes a slider connection portion that has a plate shape and is fixed to the slider, and a nut bracket connection portion that has a plate shape and is fixed to the nut bracket.

2. The actuator according to claim 1, wherein the coupling member is disposed with a clearance between the coupling member and the frame.

3. The actuator according to claim 1 or 2, wherein the coupling member has a line symmetrical shape with respect to a straight line serving as a symmetrical axis viewed from the axial direction of the screw shaft, the straight line passing through the guide rail and the screw shaft.

4. The actuator according to any one of claims 1 to 3, wherein the nut bracket includes a nut support that has a plate shape and is orthogonal to the screw shaft.

5. The actuator according to any one of claims 1 to 3, wherein the nut bracket supports the nut such that the nut is rotatable around an axis orthogonal to the axial direction of the screw shaft.

6. The actuator according to any one of claims 1 to 5, further comprising a plunger drive member that is disposed on a side opposite to the slider with the coupling member interposed therebetween and moves together with the coupling member, wherein
a length of the plunger drive member in an X direction is equal to or larger than half of a length of the coupling member in the X direction, the X direction being in parallel with a surface of the frame to which the guide rail is attached and being orthogonal to the axial direction of the screw shaft.

7. The actuator according to any one of claims 1 to 6, wherein the nut bracket connection portion is in parallel with the slider connection portion.

8. The actuator according to any one of claims 1 to 7, wherein the frame includes a base plate in contact with the guide rail and a reinforcing member disposed on a side opposite to the guide rail with the base plate interposed therebetween.
